# EUROPÄISCHE PATENTSCHRIFT

(11) **EP 1 529 538 B1**
(45) Veröffentlichungstag und Bekanntmachung des Hinweises auf die Patenterteilung: **13.12.2006**
(21) Anmeldenummer: 04025622.4
(22) Anmeldetag: 28.10.2004
(51) Int. Cl.: A61L 2/04, A23L 3/04, A23L 3/00

(54) **Pasteurisierungsanlage**
Pasteurisation apparatus
Appareil de pasteurisation

(30) Priorität: 10.11.2003 DE 10352886
(43) Veröffentlichungstag der Anmeldung: 11.05.2005
(73) Patentinhaber: KHS AG, 44143 Dortmund (DE)
(72) Erfinder: Wiedemann, Ulrich, 44135 Dortmund (DE); Münzer, Jan, 44137 Dortmund (DE)

(56) Entgegenhaltungen:
- DD-A- 230 695
- DE-B- 1 217 762
- DE-B- 1 792 383
- DE-U- 20 317 441
- US-A- 5 772 958
- US-A1- 2002 073 652
- US-A1- 2002 170 440
- US-B1- 6 374 575
- DATABASE WPI Section Ch, Week 200338 Derwent Publications Ltd., London, GB; Class D13, AN 2003-395825 XP002313312 -& ES 2 184 533 A1 (MORATA ALEMAN L) 1. April 2003 (2003-04-01)

## Beschreibung

Die Erfindung bezieht sich auf eine Pasteurisierungsanlage zum Pasteurisieren von Produkten in Behältern gemäß Oberbegriff des Anspruchs 1.

In der Getränkeindustrie ist es insbesondere bei leicht verderblichen Gütern üblich, diese zu pasteurisieren. Bei bekannten Pasteurisierungsanlagen werden dabei die Behälter mit den Produkten in praktisch gleichmäßiger Bewegung vom Eingangsbereich zum Ausgangsbereich gefördert. Während dieser Bewegung werden sie aufgeheizt, bis sie die gewünschten Pasteurisierungseinheiten aufgenommen haben, danach gekühlt, womit der Pasteurisierungsvorgang beendet ist. Ein dazu vorgesehener Pasteurisierungstunnel hat demzufolge einen Aufheizabschnitt, einen Überhitzungs- und Pasteurisierungsabschnitt mit einem nachfolgenden Abkühlabschnitt. Die einzelnen Abschnitte können weitere Unterzonen aufweisen. Die dadurch sichergestellte allmähliche Aufheizung und Abkühlung wird insbesondere bei in der Getränkeindustrie verwendeten Glasflaschen vorgezogen, um durch abrupte Temperaturänderungen ein Zerstören der Glasflaschen zu vermeiden. Die Wärmeübertragung auf das in den Behältern enthaltene Produkt geschieht normalerweise durch Besprühen dieser Behälter mit Wasser, die auf einem Förderband, welches die Sprühflüssigkeit nach unten durchtreten lässt, fortbewegt werden. Unter dem Förderband sind Auffangbehälter für die Sprühflüssigkeit angebracht, aus denen die Pumpen für die Besprühung gespeist werden. Zwischen den aufzuheizenden und abzukühlenden Abschnitten kann mit der Sprühflüssigkeit zonenweise Wärme ausgetauscht werden.

Um eine optimale Abstufung der Temperaturen in den einzelnen Abschnitten erreichen zu können, sind diese entsprechend unterteilt. Meist weist der Abschnitt "Aufheizen" drei bis vier einzelne Zonen auf, das Pasteurisierungsgebiet zwei oder drei Zonen, wobei noch eine Überhitzungszone vor der Pasteurisierungszone vorgesehen sein kann. Der anschließende Abschnitt "Abkühlen" weist wiederum drei bis vier Einzelzonen auf, in welchen die Behälter durch eine schrittweise abnehmende Temperatur der Sprühflüssigkeit bis zur gewünschten Ausgangstemperatur abgekühlt werden.

Die jeweils eingestellten Sprühtemperaturen sind genau dem Produkt, den Längen der Zonen und der Geschwindigkeit des Transporteurs angepasst, damit sichergestellt ist, dass das in den Behältern befindliche Produkt den vorgeschriebenen Pasteurisierungsgrad erreicht.

Zum Besprühen der Behälter sind in dem Pasteurgehäuse quer zur Förderrichtung eine Vielzahl nebeneinander auf Abstand gehaltene Sprührohre mit Sprühdüsen oder Sprühöffnungen und einem seitlichen Flüssigkeitszulauf vorgesehen. Sprührohre dieser Art sind beispielsweise aus der DE 297166 44 bekannt.

Ausgehend von einer solchen Pasteurisieranlage liegt der Erfindung die Aufgabe zugrunde, den bei diesen Anlagen sowie deren Pasteurgehäuse erforderlichen Herstellungs-, Montage- und Inbetriebnahmeaufwand zu reduzieren und standardisierte Gehäusemodule für vorzugsweise nahezu alle Ausführungsarten zu schaffen.

Diese der Erfindung zugrunde liegende Aufgabe wird nun dadurch gelöst, dass das Tunnelgehäuse in modularer Ausgestaltung aus einer beliebigen Anzahl von flexiblen, austauschbaren Segmenten gebildet ist, wobei diese Segmente quer oder längs zur Förderrichtung des Pasteurs verlaufen und auf parallel oder längs zu dieser Förderrichtung weisenden Zonentanks und/oder Heißwassertanks positionierbar und aneinanderreihbar sind.

Mit dieser Ausgestaltung und den in den weiteren Ansprüchen dargelegten Merkmalen sowie Beschreibungsergänzungen liegen einfache Gehäuse-Grundmodule vor, die einen hohen Gleichteileanteil aufweisen und die eine Serienfertigung vieler Komponenten zulassen. Überdies ergibt sich eine flexiblere Zonenstruktur für eine optimale Maschinenauslegung, wobei die im Wesentlichen standardisierten Gehäusemodule für nahezu alle Ausführungsarten von Wärmebehandlungsmaschinen für Behälter wie Pasteur, Rückkühler, Wärmer usw. geeignet sind.

Im Nachfolgenden wird die Erfindung anhand einer ein Ausführungsbeispiel darstellenden Zeichnung mit den Figuren 1 bis 5 näher erläutert.

In Figur 1 und der nachfolgenden Beschreibung hierzu wird zunächst auf eine allgemeine Pasteurisieranlage und deren Verfahrensabläufe Bezug genommen.

In den Figuren 2 bis 5 wird dann die entsprechende Gehäuseausbildung in Form eines Ausführungsbeispiels erläutert.

Demnach besteht die Pasteurisierungsanlage aus einem in Durchlaufrichtung der jeweiligen Behälter 1 im Anfangsbereich angeordneten Aufwärmabschnitt 2, der wiederum aus einer Vielzahl von Einzelzonen 5-7 bestehen kann, wobei die Aufwärmphase entsprechend schonend vorgenommen wird. Diesem Aufwärmabschnitt 2 schließt sich eine Überhitzungszone 8 an, dieser folgt die eigentliche Pasteurisierzone 9. Anschließend beginnt der Bereich des Abkühlabschnitts 4, der, wie auch bei den anderen Abschnitten, aus einer größeren Anzahl von Einzelzonen 10-12 bestehen kann.

Das Betriebsprogramm einer solchen Pasteurisierungsanlage wird zunächst für den Pasteurisierungsbetrieb unter optimalen Bedingungen ausgelegt. So ist beispielsweise vorgesehen, dass die erste Aufwärmzone 5 eine Besprühungstemperatur von 18°C aufweist. Entsprechend ist die Ausgangstemperatur der pasteurisierten Produkte mit einer Besprühungstemperatur von etwa 17°C vorgesehen. Die zweite Aufwärmzone 6 weist eine Besprühungstemperatur von geringfügig oberhalb 24°C auf, wobei die damit kommunizierende Abkühlzone wiederum etwas geringer bei 23°C liegen kann. Am Beispiel dieser beiden Zonen ist erkennbar, dass das Wasser aus den Zonen "Abkühlen" 4 jeweils der Zone im Abschnitt "Aufwärmen" 2 zugeleitet wird, deren gewollte Aufheiztemperatur der gewollten Abkühltemperatur am nächsten kommt. Zur Ausgleichung der jeweiligen Temperaturdifferenz wird dem Wasser aus dem Abkühlabschnitt 4 vorzugsweise Wasser aus dem ersten Tank 13 in geringfügigen Mengen zugegeben, dessen Wasser höherer Temperatur aus der letzten Station 7 des Aufwärmabschnitts 2 stammt. Im Anschluss an diesen Aufwärmabschnitt befindet sich die Überhitzungszone 8, deren Temperatur wiederum höher als die der Temperatur der letzten Aufwärmzone 7 ist und aus einem zweiten Behälter 15 mit gegenüber dem ersten Behälter 13 höhere Temperatur gespeist wird. Dem zweiten Behälter 15 ist ein dritter Behälter 16 zugeordnet, dem das Überschusswasser des Behälters 15 zugeleitet wird. Dieser dritte Behälter 16 wird zudem mittels einer Aufheizeinrichtung auf einer vorbestimmbaren höheren Temperatur gehalten. Aus diesem Behälter 16 wird auch die Überhitzungs- und Pasteurisierungszone 8, 9 beaufschlagt, wobei die ablaufende heiße Wassermenge in dem zweiten Behälter 15 gesammelt und mit dem Heißwasser des dritten Behälters 16 vermischt wird.

Bei einer auftretenden Störung, beispielsweise hervorgerufen durch einen Behälterrückstau in der Pasteurisierungsanlage, wird durch eine nicht weiter dargestellte Steuereinrichtung eine sofortige Ansteuerung bestimmter Ventile vorgenommen, so dass beispielsweise das Wasser geringerer Temperatur aus dem ersten Behälter 13 der Überhitzungs- und/oder Pasteurisierungszone 8, 9 zugeführt wird.

Den anderen Abschnitten 2, 4 kann ebenfalls Wasser geringerer Temperatur, beispielsweise einem vierten Behälter 14, zugegeben werden.

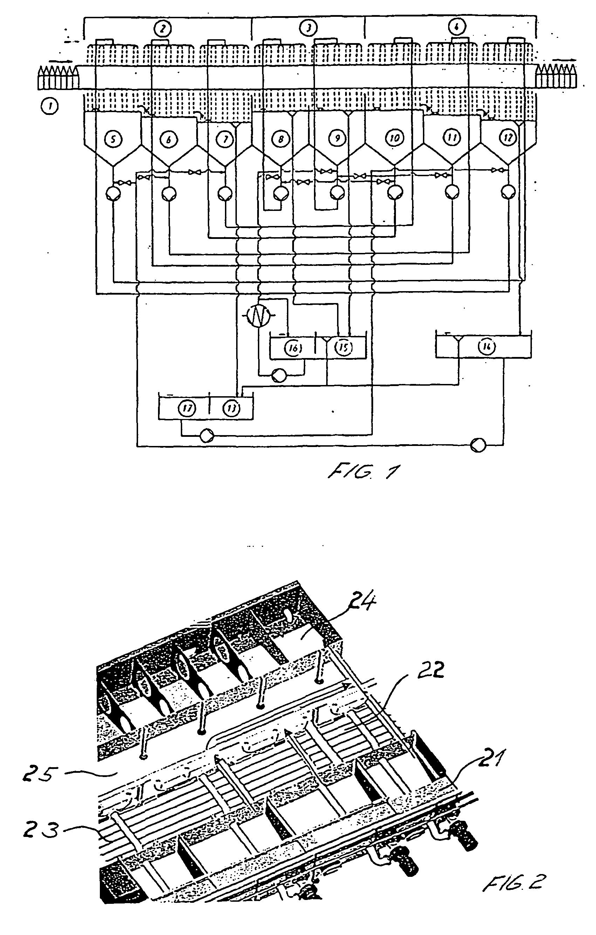

Die Vorrichtung selbst gemäß den Figuren 2 bis 5 in Form eines Pasteurgehäuses ist eine modulare Gehäusekonstruktion aus einer Vielzahl von Segmenten, wobei die Unterstruktur aus vormontierten Zonentanks 21 mit Rohrleitungen, Ventilen und Verkabelung besteht, denen seitlich die Rohrleitungen 22 in gemeinsamen oder einzelnen Segmenten zugeordnet sind. Davon auf Abstand verlaufen die Segmente 24 der Heiß-Wasser-Puffertanks. Der dazwischen verbleibende Freiraum ist als Kriechgang 25 für Wartungsfälle vorgesehen, wie Fig. 2 zeigt.

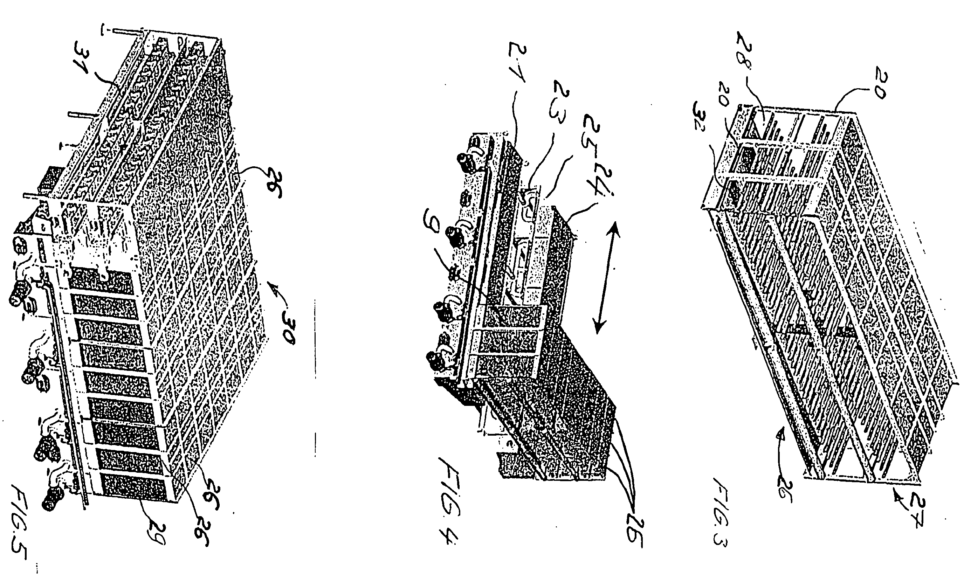

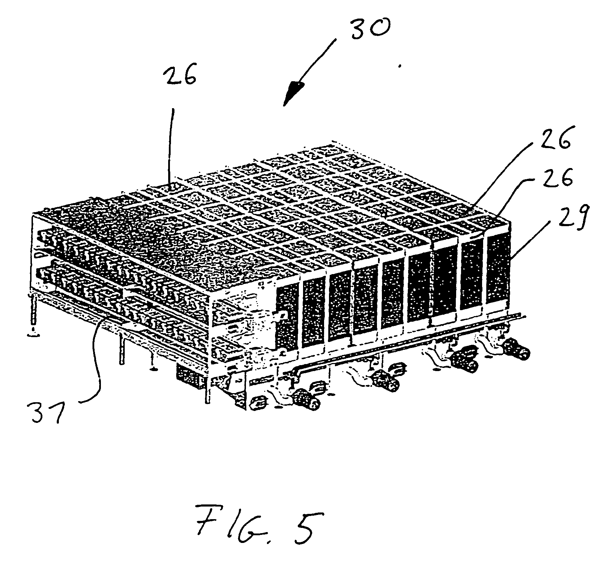

In Figur 3 ist das austauschbare und flexible Segment 6 des eigentlichen Tunnelgehäuses dargestellt. Diese einzelnen Segmente enthalten den Fördergurtunterbau mit einer Rücktrumauflage sowie die Spritzverteiler und einen Dachaufbau vorzugsweise mit Außenverrippung. Die Seitenöffnungen 28 sind mit seitlich anbringbaren Seitenpaneelen 29 abdichtbar. Die Tunnelsegmente 26 werden gemäß Figur 4 quer zur Fördervorrichtung des Pasteurs verlaufend auf den Zonentank 21 und den dazu auf Abstand befindlichen Heißwassertanks 24 aufgesetzt. Die Tunnelsegmente 26 sind im Fußbereich mit Gleitelementen, Montagerollen oder umstellbaren Gleitrollen zur vereinfachten örtlichen Verschiebung ausgestattet und in horizontaler Ebene bewegbar sowie aneinanderreihbar zu positionieren.

Auf diese Weise bilden die Tunnelsegmente 26 gemäß Figur 5 eine kompakte Gehäusestruktur 30, die durch stirnseitiges Vorschalten des eigentlichen Zu- und des eigentlichen Auslaufsegmentes 31 eine komplette, jederzeit kürzbare oder erweiterbare Pasteurisierungsanlage bilden. Dabei bildet die Maschinenbreite im Wesentlichen auch die Behandlungsbreite, wobei die kompakte Gehäusestruktur lediglich von schmalen Seitenpaneelen 29 begrenzt ist. Diese sind dichtungsfrei in den Seitenwandausbildungen 20 der Segmente 26 gehalten und zweckmäßig in einem wasserführenden unteren Rahmen 32 abgestützt. Anstelle dieser quer zur Fördervorrichtung des Pasteurs ausgerichteten Tunnelsegmente können auch Segmente verwendet werden, die parallel zur Förderrichtung aneinanderreihbar sind, wobei die Unterbauten in Form von Tanks 21 und 24 quer zu der eigentlichen Förderrichtung verlaufen.

Der Rahmen der Segmente 26 kann beispielsweise aus Rohren, Vierkantrohren oder dergleichen gebildet sein. Die Ausrüstungsteile wie Spritzrohre, Spritzverteiler, Fördergurtunterbau, Rücktrumauflage und weitere sind vorzugsweise integraler Bestandteil der Segmentkonstruktion und somit auch zur Aussteifung des Systems vorgesehen. Die standardisierten Gehäusesegmente sind in Form von Montagebaugruppen ausgebildet, wobei deren Abmessungen den erlaubten üblichen Containerinnenmaßen, z. B. eines Schiff- oder Seecontainers, entsprechen.

Die Zonentanks können zusätzlich mit Siebbandschmutz- und Scherbenförderer ausgerüstet sein. In den regenerativen Zonen ist ein kaskadierender Kühl- oder Heißwasserlauf zur optimalen Ausnutzung der Energie des Heiz- oder auch Kühlwassers vorgesehen. Die Wasserführung wird über gestufte Wehre vorgenommen, wobei das Niveau der Zonen durch geeignete Mittel wie z.B. entsprechende Sensoren und frequenzgeregelte Pumpen gehalten wird.

Die Montage der Pasteurisieranlage erfolgt vorzugsweise nach den Merkmalen des Anspruchs 15, auf den entsprechend verwiesen wird.

## Patentansprüche

1. Pasteurisieranlage zum Pasteurisieren von Produkten in Behältern, die in einem Behälterstrom durch aufeinander folgende Abschnitte mindestens zum Aufwärmen, Pasteurisieren und Abkühlen mit Hilfe von überschüttender Flüssigkeit behandelt werden sowie mit einem Pasteurgehäuse und darin angeordneten Transport-, Spritz- und anderen Einrichtungen, Wannen, Pumpen u. dgl., ***dadurch gekennzeichnet, dass*** das Tunnelgehäuse in modularer Ausgestaltung aus einer beliebigen Anzahl von flexiblen, austauschbaren Segmenten (26) gebildet ist, wobei diese Segmente (26) quer oder längs zur Förderrichtung des Pasteurs verlaufen und auf parallel oder längs zu dieser Förderrichtung weisenden Zonentanks (21) und/oder Heißwassertanks (24) aneinanderreihbar sind.

2. Pasteurisieranlage nach Anspruch 1, ***dadurch gekennzeichnet, dass*** neben den aneinandergereihten äußeren Zonentanks (21) und unterhalb der auf diesen aufgestützten Segmente (26) mindestens ein weiteres, in Segmenten vormontiertes Rohrleitungselement (23) und ein Freiraum (25) zur Wartung der unteren Bereiche vorgesehen ist.

3. Pasteurisieranlage nach den vorhergehenden Ansprüchen, ***dadurch gekennzeichnet, dass*** Zonentanks (21) in vormontierter Ausbildung mit den erforderlichen Rohrleitungen, Ventilen, Motoren, Verkabelung u. dgl. ausgestattet sind.

4. Pasteurisieranlage nach den vorhergehenden Ansprüchen, ***dadurch gekennzeichnet, dass*** den in modularer Anordnung eine kompakte Gehäusestruktur bildenden Segmenten (26) das eigentliche Ein- und das eigentliche Auslaufsegment (31) stirnseitig vorschaltbar sind.

5. Pasteurisieranlage nach den vorhergehenden Ansprüchen, ***dadurch gekennzeichnet, dass*** die Maschinenbreite im Wesentlichen auch die Behandlungsbreite bildet und die kompakte Gehäusestruktur (30) lediglich von schmalen Seitenpaneelen (29) begrenzt ist.

6. Pasteurisieranlage nach den vorhergehenden Ansprüchen, ***dadurch gekennzeichnet, dass*** die Seitenpaneelen (29) dichtungsfrei eine wasserdichte Gehäuseseitenwand bilden und mindestens im unteren Bereich in einem wasserführenden und ableitenden Rahmen (32) gehalten sind.

7. Pasteurisieranlage nach den vorhergehenden Ansprüchen, ***dadurch gekennzeichnet, dass*** die Segmente (26) mindestens eine Transportgurtunterbau, eine Rücktrumauflage, Verteiler für das Spritzwasser und einen Dachaufbau aufweisen.

8. Pasteurisieranlage nach den vorhergehenden Ansprüchen, ***dadurch gekennzeichnet, dass*** die Segmente (26) Mittel zur Vereinfachung der Montage der Segmente aufweisen.

9. Pasteurisieranlage nach Anspruch 8, ***dadurch gekennzeichnet, dass*** es sich bei diesen Mitteln um Montagerollen handelt.

10. Pasteurisieranlage nach Anspruch 8, ***dadurch gekennzeichnet, dass*** es sich bei diesen Mitteln um in ihrer Wirkrichtung umstellbare Gleitrollen handelt.

11. Pasteurisieranlage nach den vorhergehenden Ansprüchen, ***dadurch gekennzeichnet, dass*** die Segmente (26) auf den sie abstützenden Warmwasser- und/oder Zonentanks (21, 24) in Position rollbar und/oder verschiebbar geführt sind.

12. Pasteurisieranlage nach den vorhergehenden Ansprüchen, ***dadurch gekennzeichnet, dass*** der Rahmen der Segmente aus Vierkantrohren ausgebildet ist.

13. Pasteurisieranlage nach den vorhergehenden Ansprüchen, ***dadurch gekennzeichnet, dass*** die Ausrüstungsteile wie Spritzrohre u. dgl. integraler Bestandteil der Segmentkonstruktion und zur Aussteifung des Systems vorgesehen sind.

14. Pasteurisieranlage nach den vorhergehenden Ansprüchen, ***dadurch gekennzeichnet, dass*** die auf den Zonen- und/oder Heißwassertanks (21, 24) positionierten Segmente in horizontaler Ebene verschiebbar und/oder positionierbar sind.

15. Pasteurisieranlage nach den vorhergehenden Ansprüchen, ***dadurch gekennzeichnet, dass*** die standardisierten Gehäusesegmente in Form von Montagebaugruppen ausgebildet sind, deren Abmessungen den zulässigen Container-Innenmaßen entsprechen.

16. Verfahren zur Montage eines Pasteurgehäuses einer Pasteurisieranlage, ***dadurch gekennzeichnet, dass*** modularisierte Funktionsblöcke in Form von Segmenten gemäß einem vorgegebenen Plan konstruktiv koordiniert zusammengebaut werden, wobei zunächst die tragenden Bodenteile als die eigentliche Unterstruktur in Form von Tanks zusammengestellt und auf die erforderliche Breite mit einem Zwischenraum ausgerichtet und die in dem bodennahen Zwischenraum vorgesehenen Rohrleitungssegmente eingebaut werden, worauf die Segmente auf den Tanks reihenweise positioniert und die Endteile als Ein- und Auslaufgehäuse angesetzt werden, die Förderelemente eingebracht und die seitlichen Verkleidungen eingegliedert werden und die Abschlussmontage mit Inbetriebnahme vorgenommen wird.

## Claims

1. Pasteurising system for the pasteurising of products in containers that are treated in a container stream passing through consecutive sections that are provided at least for heating, pasteurising and cooling by means of liquid being poured over them, the said pasteurising system also including a pasteurising housing and conveying, spraying and other devices, tanks, pumps and the like, **characterised in that** the tunnel housing is formed in a modular manner from an arbitrary number of flexible, exchangeable segments (26), wherein the said segments (26) extend at right angles or longitudinally relative to the conveying direction of the pasteuriser and can be arranged next to one another in rows on zone tanks (21) and/or hot water tanks (24) that point parallel or longitudinally relative to the said conveying direction.

2. Pasteurising system according to claim 1, **characterised in that** at least one additional pipe line member (23), which has been pre-assembled in segments, and a free space (25) for carrying out the maintenance of the lower regions are provided next to the outer zone tanks (21), which have been arranged next to one another in rows, and underneath the segments (26) that are supported on the said tanks.

3. Pasteurising system according to the preceding claims, **characterised in that** zone tanks (21) are provided with the necessary pipe lines, valves, motors, cabling and the like in the pre-assembled embodiment.

4. Pasteurising system according to the preceding claims, **characterised in that** in a modular disposition the actual inlet segment and the actual outlet segment (31) can be connected to the segments (26) that form a compact housing structure at the end faces.

5. Pasteurising system according to the preceding claims, **characterised in that** the machine width substantially also forms the treatment width and the compact housing structure (30) is defined simply by narrow side panels (29).

6. Pasteurising system according to the preceding claims, **characterised in that** the side panels (29) form a water-tight housing side wall in a seal-free manner and are retained at least in the lower region in a frame (32) that conducts and drains water.

7. Pasteurising system according to the preceding claims, **characterised in that** the segments (26) include at least one conveyor belt substructure, a return belt support, distributing means for the spray water and a roof structure.

8. Pasteurising system according to the preceding claims, **characterised in that** the segments (26) include means to simplify the assembly of the segments.

9. Pasteurising system according to claim 8, **characterised in that** the said means are assembly rollers.

10. Pasteurising system according to claim 8, **characterised in that** the said means are sliding rollers that are adjustable in their direction of movement.

11. Pasteurising system according to the preceding claims, **characterised in that** the segments (26) are guided so as to be rollable and/or displaceable in position on the warm water tanks and/or zone tanks (21, 24) that support the said segments.

12. Pasteurising system according to the preceding claims, **characterised in that** the frame of the segments is produced from rectangular tubes.

13. Pasteurising system according to the preceding claims, **characterised in that** the equipment such as spray tubes and the like are integral components of the segment structure and are provided to give the system rigidity.

14. Pasteurising system according to the preceding claims, **characterised in that** the segments that are positioned on the zone tanks and/or hot water tanks (21, 24) are displaceable in a horizontal plane and/or are positionable.

15. Pasteurising system according to preceding claims, **characterised in that** the standardised housing segments are in the form of assembly modules, the dimensions of which correspond to the admissible inside dimensions of a container.

16. Method for assembling a pasteurising housing of a pasteurising system, **characterised in that** modularised function blocks in the form of segments are assembled together in a structurally co-ordinated manner corresponding to a predetermined plan, wherein, first of all, the bearing floor parts are put together in the form of tanks as the actual substructure and are orientated to the necessary width with an intermediate space, and the pipe line segments that are provided in the intermediate space close to the floor are installed, whereupon the segments are positioned in rows on the tanks and the end parts are mounted in the form of the inlet housing and the outlet housing, the conveying members are introduced and the side coverings are incorporated and the final assembly with commissioning is carried out.

## Revendications

1. Installation de pasteurisation pour pasteuriser des produits dans des récipients, qui sont traités dans un flux de récipient par sections consécutives au moins pour le réchauffement, la pasteurisation et le refroidissement à l'aide de liquide de recouvrement et avec un boîtier de Pasteur et des dispositifs de transport, de pulvérisationet autres dispositifs disposés à l'intérieur, tels que des cuves, des pompes, etc., **caractérisée en ce que** le boîtier tunnel est formé dans une conception modulaire à base d'un nombre quelconque de segments (26) flexibles et remplaçables, ces segments (26) étant agencés transversalement ou dans le sens longitudinal par rapport au sens de transport du Pasteur et pouvant êtrealignésles uns à côté des autres sur des réservoirs de zones (21) et/ou des réservoirs d'eau chaude (24) agencés parallèlement ou le long de ce sens de transport.

2. Installation de pasteurisation selon la revendication 1, **caractérisée en ce que**, à côté des réservoirs de zones (21) extérieurs alignés les uns à côté des autres et au-dessous des segments (26) supportéssur ce réservoir, il est prévu au moins un autre élément de conduite (23) prémonté en segments et un espace libre (25) pour la maintenance des zones inférieures.

3. Installation de pasteurisation selon les revendications précédentes, **caractérisée en ce que** des réservoirs de zones (21) sont équipé dans une réalisation prémontée avec les conduites, vannes, moteurs, câblages et similaires nécessaires.

4. Installation de pasteurisation selon les revendications précédentes, **caractérisée en ce que** le segment d'entrée proprement dit et le segment d'écoulement (31) proprement dit peuvent être montés côté avant en amont des segments (26) formant une structure de boîtier compact dans un agencement modulaire.

5. Installation de pasteurisation selon les revendications précédentes, **caractérisée en ce que** la largeur de machine forme sensiblement également la largeur de traitement et la structure de boîtier (30) compacte est limitée uniquement par des panneaux latéraux(29) étroits.

6. Installation de pasteurisation selon les revendications précédntes, **caractérisée en ce que** les panneaux latéraux (29) forment sans joint une paroi latéralede boîtier étancheà l'eau et sont maintenus au moins dans la zone inférieue dans un cadre (32) véhiculant de l'eau et évacuateur.

7. Installation de pasteurisation selon les revendications précédentes, **caractérisé en ce que** les segments (26) présentent au moins une infrastructure de sangle de transport, un support de brin retour, un répartiteur pour l'eau pulvérisée et une structure de toit.

8. Installation de pasteurisation selon les revendications précédntes, **caractérisée en ce que** les segments (26) présentent des moyens pour simplifier le montage des segments.

9. Installation de pasteurisation selon la revendication 8, **caractérisée en ce qu'**il s'agit, en ce qui concerne ces moyens, de galets de montage.

10. Installation de pasteurisation selon la revendication 8, **caractérisée en ce que**, en ce qui concerne ces moyens, il s'agit de galets de glissement pouvant être inversés dans leur sens d'effet.

11. Installation de pasteurisation selon la revendication précédente, **caractérisée en ce que** les segments (26) sont guidésde façon à rouler et/ou coulisser en position sur les réservoirsd'eau chaude et/ou de zones (21, 24) qui les soutiennent.

12. Installation de pasteurisation selon les revendications précédentes, **caractérisée en ce que** le cadre des segments est conçu à base de tubes carrés.

13. Installation de pasteurisation selon les revendications précédentes, **caractérisée en ce que** les parties d'équipement tels que des tuyaux de pulvérisation et similaires font partie intégrante de la construction par segment et sont prévues pour le renfort du système.

14. Installation de pasteurisation selon les revendications précédentes, **caractérisée en ce que** les segments positionnéssur les réservoirsde zones et sur les réservoirs d'eau chaude (21, 24) peuvent être déplacés et/ou positionnés dans un plan horizontal.

15. Installation de pasteurisation selon les revendications précédentes, **caractérisée en ce que** les segments du boîtier standardisés sont conçus sous la forme d'ensembles de montage, dont les dimensions correspondent aux cotes intérieures de conteneur autorisées

16. Procédé pour le montage d'un boîtier de Pasteur d'une installation de pasteurisation, **caractérisé en ce que** des blocs de fonction modularisés sont assemblés sous forme de segments de façon coordonnée au niveau de la construction selon un plan prédéfini d'abord les parties de fond portantes en tant que l'infrastructure proprement dite étant assemblées sous la forme de réservoirs et étant alignées sur la largeur nécessaie avec un espace intermédiaireet les segments de conduite prévusdans l'espace intermédiaie proche du fond étant montés, après quoi les segments sont positionnés rangée par rangée sur les réservoirs et les parties d'extrémitésont placées comme boîtier d'entrée et de sortie, les éléments de transport sont introduits et les habillages latéraux sont incorporéset le montage final avec la mise en service est effectué.
